# EUROPEAN PATENT APPLICATION

(11) **EP 4 230 295 A1**
(43) Date of publication of application: **23.08.2023**
(21) Application number: 22157609.3
(22) Date of filing: 18.02.2022
(51) Int. Cl.: B01L 3/00, B01L 7/00, C12Q 1/6851

(54) **ON-SITE ANALYSIS OF BIOMATERIALS**

(71) Applicant: Bühler AG, 9240 Uzwil (CH)
(72) Inventor: NAGARAJA, Hemanth, 9240 Uzwil (CH); BASHFORD, Stuart, London, E16 2BF (GB)
(74) Representative: Page White Farrer

(57) **Abstract**

Methods and apparatuses for analysing biomaterial are disclosed. In accordance with a method an onsite apparatus operable at the location of the biomaterial prepares an input sample for analysis of the biomaterial, the preparing comprising extracting deoxyribonucleic acid (DNA) from a sample of the biomaterial and processing the extracted DNA for preparing the input sample. The input sample is input into a polymerase chain reaction (PCR) component of the onsite apparatus and a PCR analysis is performed on the extracted input sample. A raw fluorescent signal is generated for the sample by the onsite apparatus based on the performed PCR analysis. The signal is used in determining initial quantities of different DNA in the input sample. The determining of initial quantities is based on a number of PCR reaction cycles of fluorescent signals of the different DNA in the raw fluorescent signal needed to cross a fluorescence threshold and reference data.

## Description

The present disclosure relates to methods, apparatuses and computer program products for analysing biomaterials. A particular example of biomaterials that can be analysed is raw materials for food products.

Food products and raw materials for food products are one of the most common biomaterials. Food materials are cultivated, treated, traded, transported, stored, and otherwise handled and processed in large quantities and various stages of food production chain. Examples of food raw materials and products comprise grain, vegetables, fruits, nuts and so on crops, oils, meat, dairy, flours, etc. A raw material for a food product can have a number varieties, come from various sources and geographical areas, be of differing quality and so on. Using rice as an example, different types of rice such as Basmati, Jasmine, Calasparra, Arborio and so forth are commonly cultivated. E.g., Basmati rice in turn comes in different varieties such as Taraori and Dehraduni. Keeping track of the type, variety, purity and composition of food materials is considered important.

Testing can be used for tracing the raw materials. Food testing procedures may require more detailed analysis than basic sensory evaluation onsite to check that a lot of the material is what it should be, is in order for further processing and ultimately can be put on general sale and consumption. Laboratory analysis may be necessary for regulatory and/or internal process control purposes. Also, raw material purchases can form a significant part of the cost of final products. Thus the ability to test composition and purity of raw materials is important as errors in raw material assessment can have significant implications on quality, cost, liability, product reputation and so on.

Testing can comprise deoxyribonucleic acid (DNA) analysis. Examples of DNA analyses include testing for genetically modified organisms (GMO), and microbiological testing for food safety and/or authentication. Currently DNA based analyses on materials such as grains and crops is in general comprise sending a sample to be tested to a laboratory which can be operated internally by the organisation or by a third-party. The analytical DNA based procedures of food materials use special equipment and skilled personnel to perform the DNA based testing. The workflow includes taking a sample onsite and thereafter a series of sample movements and precise liquid handling steps that require precision and training. The sample-to-result process can take several days. This can limit usability of the DNA based testing. For example, time critical supply chain and/or process control decisions would benefit from more efficient and faster turnaround times. Also, the quality of the results may become compromised due to the time it takes between taking a sample and getting the results from testing and analysis of the sample. Moving the sample around in non-optimal conditions may come with increased risk of contamination.

In accordance with an aspect there is provided a method of analysing biomaterial, the method comprising: preparing by an onsite apparatus operable at the location of the biomaterial an input sample for analysis of the biomaterial, the preparing comprising extracting deoxyribonucleic acid (DNA) from a sample of the biomaterial and processing the extracted DNA for preparing the input sample; introducing the input sample into a polymerase chain reaction (PCR) component of the onsite apparatus; performing a PCR analysis on the extracted input sample in the PCR component of the onsite apparatus; and generating a raw fluorescent signal for the sample by the onsite apparatus based on the performed PCR analysis for use in determining initial quantities of different DNA in the input sample, wherein the determining of initial quantities is based on a number of PCR reaction cycles of fluorescent signals of the different DNA in the raw fluorescent signal needed to cross a fluorescence threshold and reference data.

According to another aspect there is provided a method of analysing biomaterial by a data processing apparatus, the method comprising: receiving via a data communication network from an onsite apparatus operable at the location of the biomaterial a raw fluorescent signal generated for an input sample of the biomaterial, wherein the onsite apparatus has extracted deoxyribonucleic acid (DNA) from the sample and processed the extracted DNA by a polymerase chain reaction (PCR) component; and determining initial quantities of different DNA of interest in the input sample based on the received raw fluorescent signal, wherein the determining comprises determining the number of reaction cycles for fluorescent signals of the different DNA in the raw fluorescent signal needed to cross a fluorescence threshold and mapping the number to reference data.

According to another aspect there is provided an onsite apparatus for analysis of biomaterial, the apparatus comprising: equipment configured to prepare an input sample of the biomaterial by extracting deoxyribonucleic acid (DNA) from the biomaterial; a polymerase chain reaction (PCR) component configured to perform a PCR analysis on the input sample in the onsite apparatus; signal generator for generating a raw fluorescent signal for the input sample based on the PCR analysis for use in determining initial quantities of different DNA in the input sample based on determination of the number of PCR reaction cycles of fluorescent signals of the different DNA in the raw fluorescent signal needed to cross a fluorescence threshold and reference data; and data communication interface for communication of information associated with the PCR analysis.

The onsite apparatus may comprise a handheld apparatus.

In accordance with a further aspect there is provided an apparatus for analysing biomaterial, the apparatus comprising at least one processor and at least one memory including a computer program code, the at least one memory and computer program code configured to, with the at least one processor, cause the apparatus at least to: receive via a data communication network from an onsite apparatus operable at the location of the biomaterial a raw fluorescent signal generated for an input sample of the biomaterial, wherein the onsite apparatus has extracted deoxyribonucleic acid (DNA) from the sample and processed the extracted DNA by a polymerase chain reaction (PCR) component; and determine initial quantities of different DNA of interest in the input sample based on the received raw fluorescent signal by determining the number of reaction cycles for fluorescent signals of the different DNA in the raw fluorescent signal needed to cross a fluorescence threshold and mapping the number to reference data.

In accordance with a more specific aspect the raw fluorescent signal is send from the onsite apparatus to a remote data processing apparatus for the determining of initial quantities of different DNA of interest based on the number of PCR reaction cycles and reference data from a reference database. The onsite apparatus may receive from the remote data processing apparatus information of the initial quantities of different DNA of interest and/or a composition of the input sample.

In accordance with an aspect the onsite apparatus receives reference data from a reference database and determines the initial quantities based on the number of PCR reaction cycles of fluorescent signals of the different DNA in the raw fluorescent signal needed to cross the fluorescence threshold and the reference data.

The onsite apparatus may reduce particle size of the biomaterial in the sample, purify the extracted DNA, and fluidize the purified DNA.

A handheld onsite apparatus may perform the steps of receiving the sample, preparing the input sample from the received sample, performing the PCR analysis, and generating the raw fluorescent signal.

The onsite apparatus may be configured to perform in a sealed environment within the housing thereof the steps of preparing, introducing, performing and generating without operator involvement.

The PCR component may be replaceable according to the biomaterial.

Information of composition of the biomaterial may be determined based on the determined initial quantities of different DNA of interest and information of the mass of the biomaterial.

The reference data may be stored in and obtained from a database comprising information on a per DNA of interest basis that indicates a relation between the number of PCR cycles to cross the fluorescence threshold and the initial quantity of the DNA of interest in the input sample.

The data processing apparatus may signal information of the initial quantities of different DNA of interest and/or a composition to the onsite apparatus and/or a database.

The biomaterial may comprise food material. The onsite analysis may be performed in a stage of a food production chain.

An input signal may be generated for a controller of a processing system based on the results of the analysis. The processing system may be controlled at least in part based on the input signal.

A computer software product embodying at least a part of the herein described analysis functions may also be provided. In accordance with an aspect a computer program comprises instructions for causing performance of the methods described herein.

Some aspects will now be described in further detail, by way of example only, with reference to the following examples and accompanying drawings, in which:
Figure 1 illustrates a schematic example of a system comprising an onsite analysis apparatus and a remote data processing apparatus;
Figure 2 is an example of a microfluidic chip;
Figure 3 is an example of a data processor apparatus;
Figures 4 and 5 show flowcharts according to certain examples;
Figure 6 shows an example of signal curves for different DNA markers;
Figures 7 shows an example a threshold curve a DNA marker of interest; and
Figure 8 shows an example of process control arrangement.

The following description gives an exemplifying description of some possibilities to practise the invention. Although the specification may refer to "an", "one", or "some" examples or embodiment(s) in several locations of the text, this does not necessarily mean that each reference is made to the same example of embodiment(s), or that a particular feature only applies to a single example or embodiment. Single features of different examples and embodiments may also be combined to provide other embodiments.

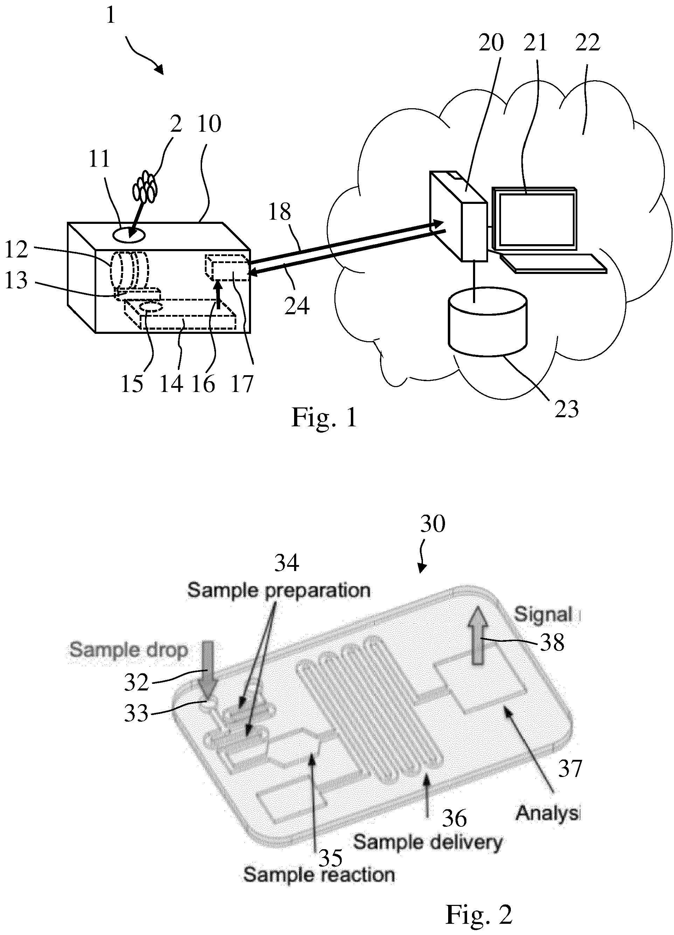

Figure 1 shows a schematic example of a testing and analysis arrangement 1 where the herein described principles can be embodied. A locally operable testing apparatus 10 provides a hardware processing platform configured to receive and process a sample 2 of biomaterial. The biomaterial can comprise, for example raw material for a food product such as grain or rice. The apparatus 10 is configured to provide a DNA based analytical procedure where polymerase chain reaction (PCR) is utilised. The apparatus can be moveable within a site and/or from site to site. The apparatus can be configured to be small and light enough to be handheld.

The apparatus 10 can be configured to receive the material 2 to be tested via an inlet opening 11. The material can be guided to a pre-processing stage where the material for the analysis can be mechanically or otherwise processed to be better suited for the DNA analysis. For example, food material can be processed by a grinder 12, a shredder or the like equipment capable of mechanically reducing food material particles into smaller pieces. The grinding, or similar process, can be arranged to crack the food material open to increase the surface area available to extract the DNA. Other preparatory actions may be taken such as drying, heating, adding agent(s), and so on. The particle size reduction may also be provided, at least in part, by non-mechanical means such as chemical agents, laser, and so forth. The pre-processing can also comprise preparing and transferring a correct amount of solid material to the next step.

In the preparation step DNA inherent in the food is released. This is often referred as extraction. After extraction, the extracted DNA is typically purified to remove PCR inhibiting factors such as proteins. The purified sample can then be fluidized before input into a reaction stage.

In Figure 1 the preparation stage is performed at a preparation equipment that is schematically presented by a single block 13. The preparation stage however may comprise a chain of separate components. In certain applications some earlier stages may be provided by equipment outside the apparatus 10.

Extracted, purified and fluidized DNA sample is introduced to a polymerase chain reaction (PCR) provided at a microfluidic chip 14. Microfluidic PCR chips are miniaturized fluidic systems for fast DNA amplification. The PCR is a molecular biological technique for addressing and exponentially amplifying a DNA fragment. The PCR process is based on temperature cycles with an enzymatic amplification step. After thermal cycling, those reaction vessels that contained target template will generate fluorescence. The microfluidic chip can have an inlet 15 for receiving the sample from the preparation equipment 13. The chip processes the sample, performs analysis and outputs signal 16. The signal can be input to a data communication unit 17.

Figure 2 shows an example of internal details of possible a microfluidic chip 30 that can be used for the local DNA analysis of the sample 2. The microfluidic chip can comprise a pattern or network of microchannels. The microchannels can be for example molded, engraved or etched in a substrate such as plastic or polymer such as PolyDimethylSiloxane, ceramics such as glass, semiconductors such as silicon and so on. The network of microchannels incorporated into the microfluidic chip is linked to the macro-environment by several holes of different dimensions hollowed out through the chip. Fluids can be injected into and evacuated from the microfluidic chip 30 through these pathways.

A precise amount of material 32 transferred from the pre-processing stage is input through inlet 33 into the chip 30.

In the example of Figure 2 a further sample preparation stage 34 is provided. At this stage an appropriate PCR agent can be introduced to the sample 32. A precise amount of the products of PCR reaction are then taken to the next step. The chip is arranged to maintain conditions considered necessary for efficient PCR (Temperature, pH).

The chip is shown to provide a sample reaction stage 35 and sample delivery stage 36. An analysis of the sample is performed after these stages by a processor component 37. An output 38 comprises a raw florescence signal that is generated for input into the data communication unit.

The microchannel network design of a microfluidic chip can be precisely elaborated to achieve desired fluid direction, mixing, separation, and/or manipulation needed in a DNA based analysis of a target material. The design of a microfluidic chip thus depends on the material to be tested and/or the purpose of the testing.

A self-sufficient microfluidic chip containing all the necessary and specific reagents for a PCR reaction can be provided as a cartridge. A hardware platform configured to accept a cartridge and run a DNA analytical procedure completely automated can provide a reduced sample-to-result time lag and reduce the need for special personnel. Different cartridges with different reagents and/or different designs can be provided for different materials to be tested and/or different tests. The cartridges can be replaceable. For example, the cartridges can be simply slotted in and out the apparatus 10. The hardware platform can thus be generic across different food and other material applications while the cartridge can be specific for each application. Replaceable cartridges are beneficial, e.g., because sample preparation reagents can be different for each application, markers per application may be different, and number and type of fluorescent agents for quantification can depend on the markers. Different reagent volumes and combination sequences may also be needed based on the application.

The cartridges can be configured based on knowledge of standard reactions of known DNA concentrations. Fluorescent raw signals based on the prior knowledge can be used to ensure that other reactions and reagents work as expected and that the results can be relied upon. Quality control can be performed by using positive and negative controls in the reaction plate itself. Certain reaction wells can contain pre-known amounts of DNA material and certain others can be without any DNA material. Addition of PCR reagents to these wells and analysis of the results can indicate if the system including PCR reactions, reagents, fluorescence detectors is working as expected.

The preparation and analysis system can be included in an insulated casing to provide a sterile, sealed and leak proof onsite apparatus to prevent contamination of the samples.

The onsite apparatus 10 can also be provided with data communication apparatus 17 arranged to communicate raw data output 16 from the microfluidic chip 14. The raw fluorescent signal 16 can be communicated via data link 18 to a remote data processing apparatus 20. The communication apparatus can be configured for wireless and/or wired communications.

The remote data processing apparatus 20 can be configured to process data from a large number of onsite analysis apparatuses. The remote data processing apparatus 20 can be provided by a cloud platform 22. The data processing apparatus 20 can comprise a user interface 21 such as a display and/or a keyboard. The data processing apparatus can comprise or be connected to a reference database 23. Analytics on the cloud platform can interpret the raw data signals 17 based on data stored in the reference database 23. For example, the remote data processing apparatus can compare received fluorescent signals against data in the reference database 23.

The apparatus 10 may also be configured for reception of data from the remote data processing apparatus 20. For example, data indicative results of further analysis, a certificate and other related information can be received from the remote data processing apparatus.

Data can be communicated based on any appropriate data communication protocol. An appropriate security protocol or protocols can be used to encrypt and/or tunnel the communications.

The data processing apparatus 20 can be comprise any suitable computer device, for example a server, configured to perform the further analysis functions as described herein. The data processing apparatus 20 can be connected to / be a part of wider data communications system, such as an Intranet or the Internet. Functions of the data processor apparatus 20 can also be at least in part provided in a cloud or virtualized environment. Appropriate gateways can be provided between different environments.

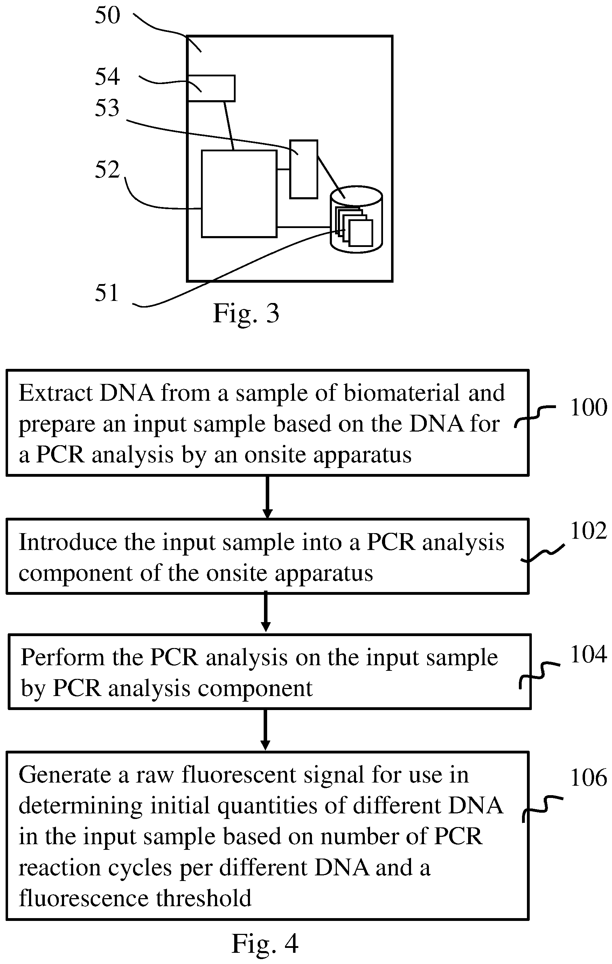

The data processing functionalities of the chip 14 and/or the data processing apparatus 20 can comprise any appropriate computing arrangement comprising at least one processor, memory, software and internal circuitry and components necessary to perform the tasks configured to implement at least some of the herein described features. Figure 3 shows an example of internal components of a data processor arrangement on a board 50 and comprising processor(s) 52, 53 and memory or memories 51. Figure 3 further shows connections between the elements of the apparatus and an interface 54 for connecting the data processing apparatus to other components. For example, in the onsite apparatus 10 of Figure the interface would be to the communication unit 17, and in the data processing apparatus 20 towards the communications system 22 and the reference database 23. The at least one memory may comprise at least one ROM and/or at least one RAM. The apparatus may comprise other possible components for use in software and hardware aided execution of tasks it is designed to perform. The at least one processor can be coupled to the at least one memory. The at least one processor may be configured to execute an appropriate software code to implement one or more of the following aspects. The software code may be stored in the at least one memory.

Figure 4 shows a flowchart according to a method of analysing biomaterial. In the method an onsite apparatus operable at the location of the biomaterial prepares at 100 an input sample for analysis of the biomaterial. The preparing comprises extracting deoxyribonucleic acid (DNA) from a sample of the biomaterial to be analysed and processing the extracted DNA for preparing the input sample. The input sample can then be introduced at 102 into a polymerase chain reaction (PCR) component of the onsite apparatus. A PCR analysis is performed at 104 on the extracted input sample in the PCR component of the onsite apparatus. A raw fluorescent signal is generated at 106 for the sample by the onsite apparatus based on the performed PCR analysis.

The raw fluorescent signal is generated for use in determining initial quantities of different DNA in the input sample. The determining may be performed by a remote data processing apparatus or at least in part by the onsite apparatus. The determining of initial quantities is based on a number of PCR reaction cycles of fluorescent signals of the different DNA in the raw fluorescent signal needed to cross a fluorescence threshold and reference data. The reference data may be obtained from a reference database. The initial quantities can be determined in various locations, e.g. in a remote server, a cloud service and/or onsite.

The generated raw fluorescent signal can be sent via a data communication link to a remote data processing apparatus for the determining of the initial quantities of different DNA of interest based on the number of PCR reaction cycles and the reference data. The remote data processing apparatus may then provide in response information of the initial quantities of different DNA of interest and/or a composition of the input sample.

According to a possibility the onsite apparatus receives reference data from a reference database and determines the initial quantities based on the number of PCR reaction cycles of fluorescent signals of the different DNA in the raw fluorescent signal needed to cross the fluorescence threshold and the reference data.

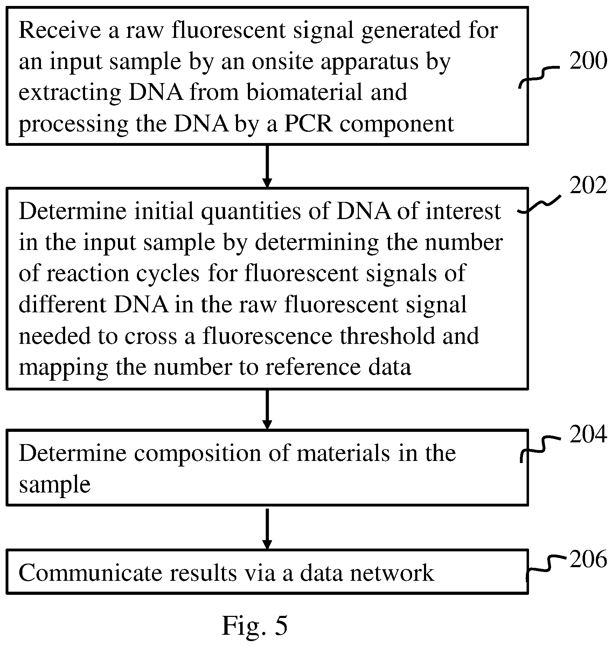

Figure 5 shows an example for operation at a remote data processing apparatus configured to receive and process raw fluorescent signals from onsite apparatus. In the method comprising a raw fluorescent signal generated for an input sample of the biomaterial is received at 200 via a data communication network from an onsite apparatus operable at the location of the biomaterial. The raw fluorescent signal has been generated for the input sample by the onsite apparatus which has extracted deoxyribonucleic acid (DNA) from the sample and processed the extracted DNA by a polymerase chain reaction (PCR) component.

Initial quantities of different DNA of interest in the input sample is determined at 202 based on the received raw fluorescent signal. The determining comprises determining the number of reaction cycles for fluorescent signals of the different DNA in the raw fluorescent signal needed to cross a fluorescence threshold and mapping the number to reference data.

Composition of materials in the sample can then be determined at 204 based on the determined initial quantities of different DNA. For example, determining information of composition of the biomaterial can be based on the determined initial quantities of different DNA of interest and information of the mass of the biomaterial.

Results of the analysis can be communicated from the data processing apparatus at 206. For example, the data processing apparatus may communicate information of the initial quantities of different DNA of interest and/or a composition to the onsite apparatus and/or a database and/or a regulator.

The preparing by the onsite apparatus can comprise at least one of reducing particle size of the biomaterial in the sample, purifying the extracted DNA, and fluidizing the purified DNA.

A handheld onsite apparatus may be configured to perform the steps of receiving the sample, preparing the input sample from the received sample, performing the PCR analysis, and generating the raw fluorescent signal.

The method may comprise performing within the onsite apparatus in a sealed environment the steps of preparing, introducing, performing and generating without operator involvement.

The reference data may be stored in and obtained from a database comprising information on a per DNA of interest basis that indicates a relation between the number of PCR cycles to cross the threshold and the initial quantity of the DNA of interest in the input sample.

The biomaterial can comprise food material and the onsite analysis is performed in a stage of a food production chain.

The result of the analysis can comprise a digital certificate containing predefined analytical data. The certificate can be communicated back to the locally operable apparatus 10. The apparatus may comprise a display for presenting the results. It is also possible to print a hard copy of the certificate. The certificate or other indication of the results can also be communicated elsewhere, for example a user device such as a smartphone or a laptop or a desktop computer of the operator of the apparatus 10, a regulatory authority and/or a database of the certificates, and so on.

The test results, such as certificates can be stored and managed by a third party service provider. The test results, such as digital certificates an also be stored in a distributed ledger such as public or private blockchain for increased integrity and trust. Instead of each party taking their own samples and performing separate testing a single test by a party is thus possible.

The workflow can be simplified from sample-to-result by automating different unit operations such as the sample preparation, DNA extraction, purification, liquid handling, polymerase chain rection (PCR), quantification, and data interpretation. The procedure can be fully automated. The preparation (e.g., drying, grinding, purification, fluidization etc) can be done completely or at least for most part by a handheld or moveable onsite device from a material that may be taken straight from the stock, carriage, bag etc. without any pre-treatment or transportation to another site. Alternatively, the preparation may be done by a separate device that is arranged co-operative with the testing device such that the prepared sample can be input substantially immediately into the onsite analysis apparatus such that contamination of the prepared samples is avoided between the preparation and testing stages.

Sample preparation may be unique for each type of raw material that is analysed. Different grinding or other particle size reducing hardware and conditions may be optimised per material. Reagents necessary for different materials can also be different. After addition of extraction reagents, the separated DNA can be filtered out of the solution, and extracted DNA alone is moved to a quantification step.

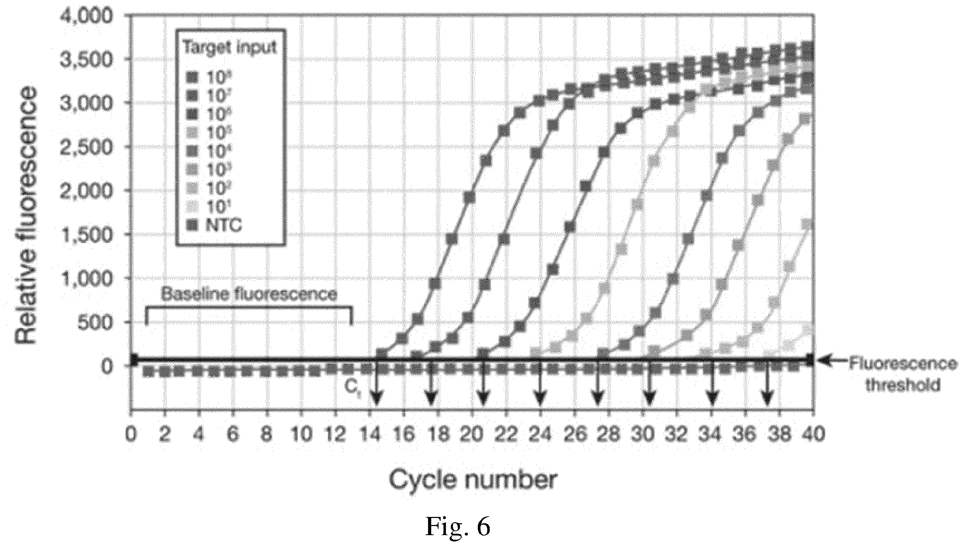

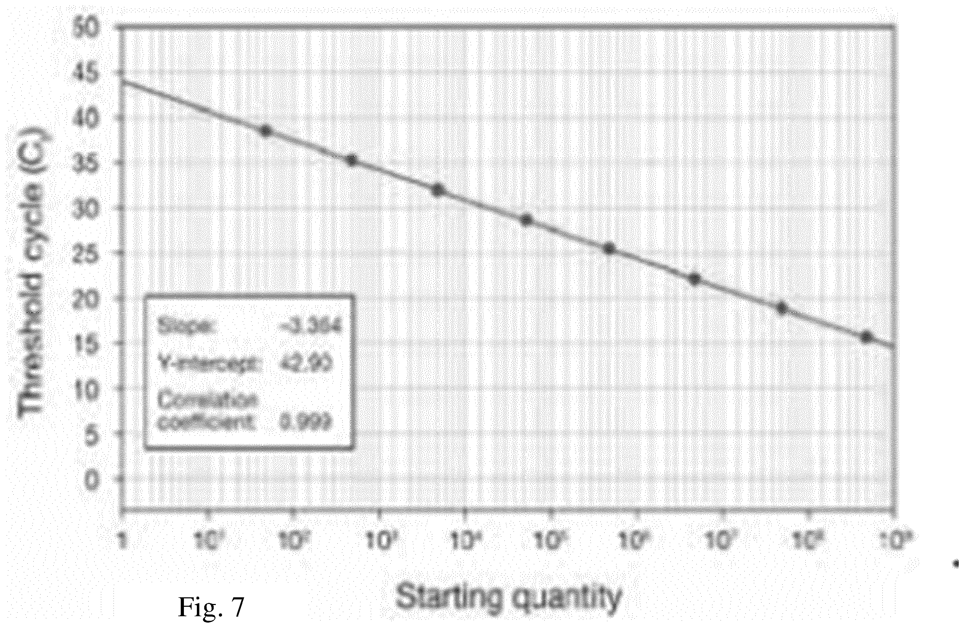

An example of analysis based on the raw fluorescence signal is explained next with reference to Figures 6 and 7. The analysis is based on fluorescence determined from the sample. The detection is based on use a beam of light that causes the electrons in the material to emit light. The light can be, e.g., ultraviolet light or a laser beam. This may be provided at the onsite apparatus.

Figure 6 shows an example of start time, slope of the signal curve and the end intensity that can be used as indicators for presence of species and quantity of species. This analysis can be based on the amplifying feature of the PCR reaction that produces millions of copies of DNA of interest. In a PCR quantification reaction, there is no apriori knowledge of the starting quantity of the DNA in the sample. As the PCR reaction progresses, the fluorescent signal follows a trajectory as shown depending on the starting amount of DNA. The cycle number at which the fluorescent signal crosses a fluorescence threshold "Ct" is proportional to amount of starting DNA.

Figure 7 shows a standard curve that can be generated beforehand and stored in the reference database for each DNA marker of interest. By comparing the information of the Ct cycles to a reference database of "Ct vs Temperature" it is possible estimate the starting quantity of DNA.

Once the starting quantity of a DNA is established, this can be correlated back to the mass of raw material to generate a percentage composition report. An example of a report based on the analysis is shown below:

**Basmati Rice Authenticity Report**

| | |
|---|---|
| Major Basmati Content | 85% |
| Major Basmati Variety | Taraori |
| Minor Basmati Content | 12% |
| Minor Basmati Variety | Dehradun |
| Riced content not approved as Basmati | 3% |

In other words, comparisons between the reference data and the raw DNA data comprise two correlations per marker of interest. Ct to cycle number gives the starting amount of DNA. Then starting DNA material can be mapped to percentage composition in the sample.

The data processing device 20 can run an algorithm generating the percentage composition starting from raw fluorescent data received from the onsite apparatus 10. The system can be completely autonomous once the sample is introduced into the system through the onsite apparatus. The sample and data derived therefrom can loop through different steps automatically without a possibility of human error in liquid transfer or missed process or analysis step.

In accordance with an example onsite food testing procedure and equipment is configured to enable a testing workflow where an operator can introduce a sample to be tested for an onsite operable device. The operator may have first inserted a specific cartridge to a generic hardware of the device according to the test requirements. The hardware then proceeds to prepare the sample (e.g., grinds, smashes, or otherwise breaks the material into smaller pieces). The device can then extract and purify the DNA, perform a PCR procedure and quantify the result in an automated manner. The raw signal from quantification is communicated to an online cloud platform where further data analytics is performed to generate the sample specific analytical result.

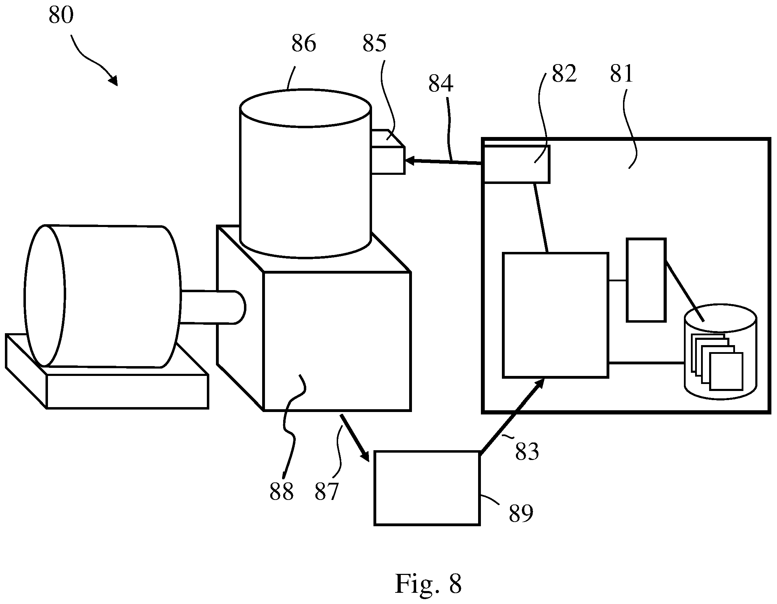

In accordance with an example schematically shown in Figure 8 outcome of the analysis can be used in controlling at least a part of a delivery and/or manufacturing process. For example, a raw material blending process may be adjusted. This can be beneficial, e.g., where a product delivery contract has been set based on an agreed composition of grain varieties at the end product. In Figure 8 example a production system 80 comprises a processing apparatus, e.g., a milling and/or blending apparatus 88. A raw material input chute 86 is controlled by a controller 85. The controller is configured to adjust the relative amount of different material, i.e., the blend of the material fed in in the processing apparatus 88. The control is based at least in part of a control signal 84 from data processing apparatus configured to perform the above described PCR based raw material analysis by an onsite apparatus. The analysis apparatus 81 generates signal 84 based on the raw fluorescence signal 83 from onsite testing by onsite apparatus 89 of the output 87 of the process by apparatus 88. The signal 84 is transmitted via interface 82 to the controller 85 of the processing apparatus based on the results of the analysis. The analysis apparatus 81 can be provided by an onsite apparatus or by a remote data processing apparatus as described above. The feeding of the processing apparatus can be controlled at least in part based on the closed loop input signal 84.

For example, mills or other operations blending, packaging and so forth food materials can adjust rapidly blending of different varieties based on a sample. The herein described DNA based method to quantify composition can be used remove a source of error in the composition. The blends can be based well to the specifications based on relative rapid analysis and without laboratory testing. The result may be input into the blending control system. Similarly, e.g., when packaging of other food products substantially rapid and accurate feedback data regarding the compositions of the raw materials may be provided. An end product certificate of authenticity may be provided efficiently and rapidly.

In case of food safety testing the sample may need to be cultured for a period of time. This may be needed so that the microbes are allowed to multiply under constant temperature conditions. An appropriate period for some samples may be, for example, 24h. The onsite apparatus can be configured to automatically control this part of the preparations of the sample.

Efficient and rapid sample-to-result workflow may be provided. Errors due to spiled or contaminated samples and/or handling and laboratory errors may be reduced. Raw material procurement and/or pricing may be determined based on the varietal purity and homogeneity of the lots. A subjective, DNA based authentication of raw materials can be used to remove a source of error in procurement of raw materials. The test data may be used for other value chain transparency products such as supplier benchmarking, commodity tracking, collaboration with regulators and consumers.

A digital platform can be arranged to collaborate between various parties to reduce the overall testing costs and testing equipment. For example, buyers and sellers of a product can avoid performing similar tests to confirm quality, and instead refer to the database where result of testing by one of the parties is available.

It is noted that although the above detailed examples have been described with reference to certain applications there are several variations and modifications which may be made to the disclosed solution without departing from the scope of the present invention. In particular, the different embodiments have been described as examples. Different features from different embodiments may be combined.

Specific software can be provided for the control and quantification of the DNA. Continuous online monitoring of the onsite apparatus, for example its working conditions, health and calibration may be provided.

In general, some embodiments may be implemented in hardware or special purpose circuits, software, logic or any combination thereof. For example, some aspects of the apparatus may be implemented in hardware, while other aspects may be implemented in firmware or software which may be executed by a controller, microprocessor or other computing device, although embodiments are not limited thereto. While various embodiments may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

The embodiments may be implemented by computer software stored in a memory and executable by at least one data processor or by hardware, or by a combination of software and hardware. Further in this regard it should be noted that any of the above procedures may represent program steps, or interconnected logic circuits, blocks and functions, or a combination of program steps and logic circuits, blocks and functions. The software may be stored on such physical media as memory chips, or memory blocks implemented within the processor, magnetic media such as hard disk or floppy disks, and optical media such as for example DVD and the data variants thereof, CD. The memory may be of any type suitable to the local technical environment and may be implemented using any suitable data storage technology, such as semiconductor based memory devices, magnetic memory devices and systems, optical memory devices and systems, fixed memory and removable memory. The data processors may be of any type suitable to the local technical environment, and may include one or more of general purpose computers, special purpose computers, microprocessors, digital signal processors (DSPs), application specific integrated circuits (ASIC), gate level circuits and processors based on multi core processor architecture, as non-limiting examples. While certain aspects of the invention may be illustrated and described as block diagrams, flow charts, or using some other schematic pictorial representation, it is well understood that these blocks, apparatus, systems, techniques and methods described herein may be implemented at least in part in, as non-limiting examples, hardware, software, firmware, special purpose circuits or logic, general purpose hardware or controller or other computing devices, or some combination thereof.

The foregoing description provides by way of exemplary and non-limiting examples a full and informative description of exemplary embodiments of the invention. However, various modifications and adaptations may become apparent to those skilled in the relevant arts in view of the foregoing description, when read in conjunction with the accompanying drawings and the appended claims. All such and similar modifications of the teachings of this invention will still fall within the spirit and scope of this invention.

## Claims

1. A method of analysing biomaterial, the method comprising:
preparing by an onsite apparatus operable at the location of the biomaterial an input sample for analysis of the biomaterial, the preparing comprising extracting deoxyribonucleic acid (DNA) from a sample of the biomaterial and processing the extracted DNA for preparing the input sample;
introducing the input sample into a polymerase chain reaction (PCR) component of the onsite apparatus;
performing a PCR analysis on the extracted input sample in the PCR component of the onsite apparatus; and
generating a raw fluorescent signal for the sample by the onsite apparatus based on the performed PCR analysis for use in determining initial quantities of different DNA in the input sample, wherein the determining of initial quantities is based on a number of PCR reaction cycles of fluorescent signals of the different DNA in the raw fluorescent signal needed to cross a fluorescence threshold and reference data.

2. The method according to claim 1, comprising
sending the raw fluorescent signal to a remote data processing apparatus for the determining of initial quantities of different DNA of interest based on the number of PCR reaction cycles and reference data from a reference database, and/or
receiving from the remote data processing apparatus information of the initial quantities of different DNA of interest and/or a composition of the input sample.

3. The method according to claim 1, comprising the onsite apparatus receiving the reference data from a reference database and determining the initial quantities based on the number of PCR reaction cycles of fluorescent signals of the different DNA in the raw fluorescent signal needed to cross the fluorescence threshold and the reference data.

4. The method according to any preceding claim, wherein at least one of
the preparing by the onsite apparatus comprises at least one of reducing particle size of the biomaterial in the sample, purifying the extracted DNA, and fluidizing the purified DNA,
a handheld onsite apparatus performs the steps of receiving the sample, preparing the input sample from the received sample, performing the PCR analysis, and generating the raw fluorescent signal,
the onsite apparatus performs in a sealed environment within the housing thereof the steps of preparing, introducing, performing and generating without operator involvement, and/or
the PCR component is replaced according to the biomaterial.

5. A method of analysing biomaterial by a data processing apparatus, the method comprising:
receiving via a data communication network from an onsite apparatus operable at the location of the biomaterial a raw fluorescent signal generated for an input sample of the biomaterial, wherein the onsite apparatus has extracted deoxyribonucleic acid (DNA) from the sample and processed the extracted DNA by a polymerase chain reaction (PCR) component; and
determining initial quantities of different DNA of interest in the input sample based on the received raw fluorescent signal, wherein the determining comprises determining the number of reaction cycles for fluorescent signals of the different DNA in the raw fluorescent signal needed to cross a fluorescence threshold and mapping the number to reference data.

6. The method according to any preceding claim, comprising determining information of composition of the biomaterial based on the determined initial quantities of different DNA of interest and information of the mass of the biomaterial.

7. The method according to any preceding claim, comprising obtaining the reference data from a database comprising information on a per DNA of interest basis that indicates a relation between the number of PCR cycles to cross the fluorescence threshold and the initial quantity of the DNA of interest in the input sample.

8. The method according to claim 5 or any claim dependent on claim 5, comprising sending from the data processing apparatus information of the initial quantities of different DNA of interest and/or a composition to the onsite apparatus and/or a database.

9. The method according to any preceding claim, wherein the biomaterial comprises food material and the onsite analysis is performed in a stage of a food production chain.

10. The method according to any preceding claim, comprising generating an input signal for a controller of a processing system based on the results of the analysis and controlling the processing system at least in part based on the input signal.

11. An onsite apparatus for analysis of biomaterial, the apparatus comprising:
equipment configured to prepare an input sample of the biomaterial by extracting deoxyribonucleic acid (DNA) from the biomaterial;
a polymerase chain reaction (PCR) component configured to perform a PCR analysis on the input sample in the onsite apparatus;
signal generator for generating a raw fluorescent signal for the input sample based on the PCR analysis for use in determining initial quantities of different DNA in the input sample based on determination of the number of PCR reaction cycles of fluorescent signals of the different DNA in the raw fluorescent signal needed to cross a fluorescence threshold and reference data; and
data communication interface for communication of information associated with the PCR analysis.

12. An apparatus for analysing biomaterial, the apparatus comprising at least one processor and at least one memory including a computer program code, the at least one memory and computer program code configured to, with the at least one processor, cause the apparatus at least to:
receive via a data communication network from an onsite apparatus operable at the location of the biomaterial a raw fluorescent signal generated for an input sample of the biomaterial, wherein the onsite apparatus has extracted deoxyribonucleic acid (DNA) from the sample and processed the extracted DNA by a polymerase chain reaction (PCR) component; and
determine initial quantities of different DNA of interest in the input sample based on the received raw fluorescent signal by determining the number of reaction cycles for fluorescent signals of the different DNA in the raw fluorescent signal needed to cross a fluorescence threshold and mapping the number to reference data.

13. The apparatus of claim 11 or 12, comprising an interface for
sending or receiving the raw fluorescent signal for the determining of initial quantities of different DNA of interest based on the number of PCR reaction cycles and reference data from a reference database, and/or
sending or receiving information of the initial quantities of different DNA of interest and/or a composition of the input sample, and/or
receiving the reference data from a reference database, the apparatus being configured for determining the initial quantities based on the number of PCR reaction cycles of fluorescent signals of the different DNA in the raw fluorescent signal needed to cross the fluorescence threshold and the reference data.

14. The apparatus according to any of claims 11 to 13, wherein the onsite apparatus is configured to at least one of reduce particle size of the biomaterial in the sample, purify the extracted DNA, fluidize the purified DNA, and generate the raw fluorescent signal without operator involvement and/or wherein the on-site apparatus comprises a handheld device configured to receive the sample, prepare the input sample from the received sample, perform the PCR analysis, generate the raw fluorescent signal, provide a sealed environment within the housing thereof, and perform the PCR analysis, and/or wherein the PCR component is replaceable according to the biomaterial.

15. The apparatus according to any of claims 11 to 14, configured to
determine information of composition of the biomaterial based on the determined initial quantities of different DNA of interest and information of the mass of the biomaterial, and/or
obtain the reference data from a database comprising information on a per DNA of interest basis that indicates a relation between the number of PCR cycles to cross the fluorescence threshold and the initial quantity of the DNA of interest in the input sample.
